# EUROPEAN PATENT APPLICATION

(11) **EP 2 399 452 A2**
(43) Date of publication of application: **28.12.2011**
(21) Application number: 10743984.6
(22) Date of filing: 23.02.2010
(51) Int. Cl.: A01H 1/00, C12N 15/29, C12N 15/63

(54) **METHOD FOR PRODUCING PLANTS WITH IMPROVED OR SUPPRESSED BLUE LIGHT RECOGNITION CAPABILITIES**

(30) Priority: 23.02.2009 KR 20090014882
(71) Applicant: Postech Academy-industry Foundation, Gyeongsangbuk-do 790-784 (KR)
(72) Inventor: NAM, Hong Gil, Pohang-si Gyeongbuk 790-751 (KR); KIM, Hyo Jung, Phohang-si Gyeongbuk 790-784 (KR); JUNG, Soo Young, Uiwang-si Gyeonggi-do 437-770 (KR); LEE, Dong Hee, Busan 614-790 (KR); HONG, Sung Hyun, Pohang-si Gyeongbuk 790-330 (KR); CHOI, Hyunmo, Jecheon-si Chungbuk 390-080 (KR)
(74) Representative: Betten & Resch
(86) International application number: PCT/KR2010/001111
(87) International publication number: WO 2010/095909

(57) **Abstract**

The resent invention relates to a method for producing plants with improved or suppressed blue light recognition capabilities.

## Description

### [Invention Title]

### METHOD FOR PRODUCING PLANTS WITH IMPROVED OR SUPPRESSED BLUE LIGHT RECOGNITION CAPABILITIES

### [Technical Field]

The present invention relates to a method for producing a plant with enhanced or suppressed blue light recognition capability.

### [Background Art]

Light, the sole source of energy and an environmental factor, plays a critical role in the growth and development of plants. To respond to light, plants have evolved various kinds of photoreceptors that accurately perceive light signals depending on their wavelength, intensity and direction.

There are many photoreceptors known to mediate light perception, including phytochromes that is sensitive to light in the red and far-red region of the visible spectrum (Botto et al. 1996; Chory et al. 1996), cryptochromes that perceive blue light (Ahmad et al. 1998; Christie et al. 1998; Cashmore et al. 1997) and UV A/B photoreceptor for UV light reception (Christie et al. 1996).

As plant photoreceptors recognizing light in the red and far-red portion of the spectrum, phytochromes are involved in various physiological responses including the germination of seeds, the development of seedlings, efflorescence, etc. Cryptochromes, found in all organisms including bacteria, plants and animals, are adapted to perceive blue light, and play similar roles to those of Phytochromes in plants.

Light perception ability is very important to plants, especially crops because it is directly associated with the productivity of crops. Plants with higher light perception ability can produce greater crops given the same quantity of light. For this reason, those engaged in plant engineering have made efforts to control light perception ability in plants by genetic manipulation.

The present invention is achieved in the context of this background.

### [Disclosure]

### [Technical Problem]

It is an object of the present invention to provide a method for producing a plant with enhanced or suppressed blue light perception ability.

Other objects and concrete embodiments of the present invention wall be given, below.

### [Technical Solution]

In accordance with an aspect thereof, the present invention pertains to a method for producing a plant having an enhanced blue light perception ability.

The method for producing a plant having enhanced blue light perception ability in accordance with the present invention comprises (I) overexpressing a gene having the nucleotide sequence of SEQ ID NO: 1 or a nucleotide sequence similar to that of SEQ ID NO: 1, and (II) selecting the plant which is enhanced in blue light perception ability as a result of the overexpression of the gene.

As used herein, the term "overexpression" in all its grammatical forms and spelling variations means a gene expression at a level higher than that in a wild-type plant when the same culture conditions such as temperature, the period of time of light and dark, etc. are given.

As used herein, the phase "gene having a nucleotide sequence similar to that of SEQ ID NO: 1" is intended to refer to a homologue of the gene of the nucleotide sequence of SEQ ID NO: 1 which encompasses all genes that have different nucleotide sequences according to plant species as a result of different evolution paths in conjunction with blue light perception function. A gene which shares higher homology with the nucleotide sequence of SEQ ID NO: 1 is more preferable. Of course, a sequence homology of 100% is the most preferable. As for the lower limit of the homology with the nucleotide sequence of SEQ ID NO: 1, it is preferably 60%. In more detail, more preferable are sequence homologies of 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99%, in ascending order of preference.

Another preferred example of the gene having a nucleotide sequence similar to that of SEQ ID NO: 1 is a functional equivalent to the gene which arises as a consequence of codon degeneracy. The functional equivalent of the gene encompasses polynucleotides coding for a polypeptide having the amino acid sequence of SEQ ID NO: 2.

In the method of the present invention, the overexpression step (I) preferably comprises transforming a polynucleotide coding for the amino acid sequence of SEQ ID NO: 2, particularly a polynucleotide having the nucleotide sequence of SEQ ID NO: 1 into a plant.

The term "transformation" in all its grammatical forms and spelling variations, as used herein, means the introduction of a foreign polynucleotide (encoding a polypeptide able to perceive blue light) into a host plant and more accurately into a plant cell irrespective of how, resulting in a genetic alteration in the host plant. Once introduced into a host plant, the foreign polynucleotide may remain integrated into the genome of the host plant or be separated, both being encompassed by the present invention.

The transformation of a plant with a foreign polynucleotide may be achieved using typical methods known in the art (Methods of Enzymology, Vol, 153, 1987, Wu and Grossman ed., Academic Press).

For the transformation, a foreign polynucleotide may be inserted into a vector, such as a plasmid or a virus, which is used as a vehicle carrying the foreign polynucleotide to plants. Further, the recombinant vector may be transformed into Agrobacterium bacteria which is used as a mediator for DNA transmission to plants (Chilton et al., 1977, Cell 11:263:271). Alternatively, a foreign polynucleotide may be introduced directly into a plant (Lorz et al., 1985, Mol. Genet. 199:178-182).

Thanks to its excellent DNA transmission capability, Agrobacterium tumefaciens is the most widely used agent for delivering a foreign polynucleotide into plants, e.g., plantlets, plant cells, seeds, adult plants, When cultured under the proper conditions, the plants, such as plantlets, plant cells, seeds, etc. after being transected with the bacteria, may grow to adult plants.

The transformation step may be carried out by (a) constructing a recombinant expression vector in which a polynucleotide coding for a polypeptide having the amino acid sequence of SEQ ID NO: 2 is operably linked to a regulatory nucleotide sequence, and (b) introducing the recombinant vector into a plant.

More preferably, the transformation step comprises constructing a recombinant expression vector in which a polynucleotide coding for the polypeptide of the present invention is operably linked to a regulatory nucleotide sequence, transforming the recombinant expression vector into an Agrobacterium species, and transfecting the Agrobacterium species into the plant. Preferably, the transformed Agrobacterium species is Agrobacterium tumefaciens.

The term "regulatory nucleotide sequence," as used herein, is intended to include any sequence that has an influence on the expression of a gene linked thereto. Examples of the regulatory nucleotide sequence include a leader sequence, an enhancer, a promoter, an initiation codon, a termination codon, a replication origin, a ribosomal binding site, etc.

The term "operably linked," as used herein, means a functional linkage between a gene of interest and a regulatory nucleotide sequence such that the transcription and/or translation of the gene is affected by the regulatory nucleotide sequence. For example, if a promoter affects the transcription of a gene of interest located in the same vector, the gene is operably linked.

In an expression vector, a promoter may be inducible or constitutive. Representative among the constitutive promoters are a CaMV promoter, a CsVMV promoter and an Nos promoter. Examples of the inducible promoter (its activity is induced by the presence of an inducer so as to allow the transcription and expression of a transgene linked thereto to be transcribed and expressed) include copper-responsive yeast metallothionein promoters (Mett et al., Proc. Natl. Acad. Sci., U.S.A., 90:4567, 1993), substituted benzenesulfonamide-inducible In2-1 and In2-2 promoters (Hershey et al., Plant Mol. Biol., 17:679, 1991), glucocorticoid response elements (GRE) (Schena et al., Proc. Natl. Acad. Sci., U.S.A., 88:10421, 1991), ethanol-responsive promoters (Caddick et al., Nature Biotech., 16:177, 1998), light-inducible promoters from the small subunit of ribulose bisphosphate carboxylase (ssRUBISCO) (Coruzzi et al., EMBO J., 3:1671, 1984; Broglie et al., Science, 224:838, 1984), mannopine synthase promoters (Velten et al., EMBO J., 3:2723, 1984), nophaline synthase (NOS) and octopine synthase (OCS) promoters, and heat-shock promoters (Gurley et al., Mol. Cell. Biol., 6:559, 1986; Severin et al., Plant Mol. Biol., 15:827, 1990).

The recombinant vector may contain a marker gene for selection. The term "marker gene," as used herein, refers to a gene encoding a phenotype that allows the selection of the transformant anchoring the maker gene. The market gene may be, for example, a gene resistant to an antibiotic or a herbicide. Suitable among the selection marker gene are an adenosine deaminase gene, a dehydrofolate reductase gene, a hygromycin-B-phosphotransferase, a thymidine kinase gene, a xanthin-guanine phosphoribosyltransferase gene, and a phosphinotricin acetyltransferase gene.

In one embodiment of the present invention, a gene having the nucleotide sequence of SEQ ID NO: 1 is inserted into pCsVMV-GFP to construct the recombinant vector pCsVMV-GFP::BIT1 which was transformed info Agrobacterium tumefaciens which was in turn infected into Arabidopsis thaliana.

In accordance with an embodiment, the transformation step is preferably carried out by introducing the gene having the nucleotide sequence of SEQ ID NO: 1 into a plant, more preferably by introducing a recombinant vector carrying the gene, particularly, the recombinant vector pCsVMV-GFP::BIT1 into a plant, and most preferably by transfecting the Agrobacterium tumefaciens transformed with the recombinant vector, especially pCsVMV-GFP::BIT1, into a plant.

In the method of the present invention, the selection step (II) may be carried out by culturing the plant under a blue light source and comparing lengths of the hypocotyls thereof with the naked eye, or by taking advantage of the selection marker when the marker gene is transformed together with it. Optionally, as will be illustrated in the following examples, the selection of transformed plants may be accomplished by comparing the degree of accumulation of anthocyanin or by determining the overexpression of the gene of interest. As described previously [Chentao Lin et al., Proc. Natl. Acad. Sci. USA Vol. 95, pp. 2686-2690, 1998] (which is incorporated herein by reference in its entirety), it is demonstrated in the following Example that when a plant is enhanced in blue light perception ability, the growth of its hypocotyls is suppressed.

In accordance with anther aspect thereof, the present invention pertains to a method for producing a plant having suppressed blue light perception ability.

The method for producing a plant having suppressed blue light perception ability in accordance with the present invention comprises (I) down-regulating expression of a gene having the nucleotide sequence of SEQ ID NO: 1 or a nucleotide sequence similar to that of SEQ ID NO: 1, and (II) selecting the plant which shows suppressed blue light perception ability as a result of the down-regulation.

As for the gene having a nucleotide sequence similar to that of SEQ ID NO: 1, its meaning and preferable embodiments are as described above.

In this method, the down-regulation step (I) may comprise transforming the plant with an antisense nucleotide sequence complementary to the gene having the nucleotide sequence of SEQ ID NO: 1 or its mRNA or to the gene having a nucleotide sequence similar to that of SEQ ID NO: 1 or its mRNA.

The antisense nucleotide sequence is intended to refer to a poly- (or oligo-) nucleotide that binds complementarily to the gene having the nucleotide sequence of SEQ ID NO: 1 or the gene having a nucleotide sequence similar to that of SEQ ID NO: 1 to interfere with the transcription or translation of the gene.

So long as the antisense nucleotide sequence interferes with the transcription or translation of the gene by binding to the gene or its transcript, no particular limitations are imparted to the length or the complementarity of the antisense nucleotide. For example, when it is 100% complementary to a gene of interest (inclusive of DNA and RNA), a polynucleotide, although as short as 30 nucleotides long, can act as an antisense nucleotide sequence of the present invention if it is subjected to suitable conditions, e.g., concentration or pH. On the other hand, when it is long enough to interfere with the transcription or translation of the gene, a polynucleotide, although not showing 100% complementarity to the gene, can act as an antisense nucleotide sequence of the present invention. Therefore, all antisense nucleotide sequences that can interfere with the transcription or translation of the gene irrespective of their length and complementarity to the gene of interest fall within the scope of the present invention. On the basis of the sequences of SEQ ID NOS: 1 and 2, the length and complementarity which are required for the use of a polynucleotide as an antisense nucleotide sequence, and the preparation of the antisense nucleotide sequence can be determined by those skilled in the art.

Preferably, the antisense nucleotide sequence comprises a region complementary to a part of the nucleotide sequence of SEQ 1D NO: 1. The phrase "a region complementary to a part of the nucleotide sequence of SEQ ID NO: 1," as used herein, means that the antisense nucleotide sequence is long enough to complementarity bind to the DNA having the nucleotide sequence of SEQ ID NO: 1 or its transcript to interfere with the transcription or translation of the gene or the transcript.

The introduction of an antisense nucleotide sequence into a plant may be typically carried out by constricting an expression vector carrying the antisense nucleotide sequence, transforming the expression vector into Agrobacterium bacteria and transfecting the transformed Agrobacterium bacteria into the plant.

Also, a further description of the transformation of the antisense nucleotide sequence into a plant may resort to that given to the method for producing a plant having an enhanced ability to perceive blue light.

In the method, the down-regulation step (I) may be achieved using a technique well known in the art. For example, gene deletion, gene insertion, T-DNA introduction, homologous recombination or transposion tagging, or siRNA (small interfering RNA) may be employed.

As in the method for producing a plant having an enhanced blue light perception ability, the selection step (II) of the method for producing a plant having a suppressed blue light perception ability may be carried out by comparing hypocotyl lengths, the degree of accumulation of anthocyanin, or the degree of suppression of gene expression, or by taking advantage of the selection marker.

In accordance with a further aspect thereof, the present invention pertains to a plant having an enhanced or suppressed ability to perceive blue light, produced by the method of the present invention.

In accordance wish still a further aspect thereof, the present invention pertains to a method for producing an anthocyanin-hyperaccumulated plant.

The method for producing an anthocyanin-hyperaccumulated plant in accordance with the present invention comprises (I) overexpressing a gene having the nucleotide sequence of SEQ ID NO: 1 or a nucleotide sequence similar to that of SEQ ID NO: 1, and (II) selecting the plant in which anthocyanin is hyperaccumulated as a result of the overexpression of the gene.

As used herein, the term "hyperaccumulation" in all its grammatical forms and spelling variations means a level of accumulation higher than that of a wild-type plant when the same culture conditions such as temperature, the period of time of light and dark, etc. are provided.

As for the description of the gene having a nucleotide sequence similar to that of SEQ ID NO: 1 and the preferred embodiment of steps (I) and (II) in the method for producing an anthocyanin-hyperaccumulated plant, their definitions given in the method for producing a plant having an enhanced blue light perception ability may be used.

In accordance with still anther aspect thereof, the present invention pertains to a method for producing an anthocyanin-hypoaccumulated plant.

The method for producing an anthocyanin-hypoaccumulated plant in accordance with the present invention comprises: (1) down-regulating the expression of a gene having the nucleotide sequence of SEQ ID NO: 1 or a nucleotide sequence similar to that of SEQ ID NO: 1, and (II) selecting the plant which shows the hypoaccumulation of anthocyanin as a result of the down-regulation.

As, used herein, the term "hypoaccumulation" in all its grammatical forms and spelling variations means a level of accumulation lower than that of a wild-type plant when the same culture condition such as temperature, the period of time of light and dark, etc. are provided.

As for the description of the gene having a nucleotide sequence similar to that of SEQ ID NO: 1 and the preferred embodiment of steps (I) and (II) in the method for producing an anthocyanin-hypoaccumulated plant, their definition given in the method for producing a plant having a suppressed blue light perception ability may be used.

In accordance with yet a further aspect thereof, the present invention pertains to a method for selecting a transformed plant using a polynucleotide having the nucleotide sequence of SEQ ID NO: 1 as a marker.

The method for selecting a transformed plant in accordance with the present invention comprises (I) transforming a plant with an expression vector containing a target gene, a polynucleotide having the nucleotide sequence of SEQ ID NO: 1 and a regulatory nucleotide sequence, said nucleotide sequence of SEQ ID NO:1 encoding a protein causative of the hyperaccumulation of anthocyanin, and (II) selecting a plant in which anthocyanin is hyperaccumulated.

The term "target gene," as used herein, means a gene to be expressed, having a polynucleotide sequence encoding a desired product (e.g., RNA or polypeptide). The polynucleotide sequence may take a truncated form, a fusion form or a tagged form and may be cDNA or gDNA encoding a native product or a desired mutant.

In this method, the transformation step (I) may comprise transforming the expression vector into Agrobacterium bacteria and transfecting the transformed Agrobacterium bacteria into the plant. The Agrobacterium bacteria is preferably Agrobacterium tumefaciens.

In the selection step (II), the hyperaccumulation of anthocyanin may be determined with the naked eye. If the determination is impossible with the naked eye, anthocyanin may be isolated and quantified using a technique known in the art.

The methods of the present invention may be applied to any plant that has photoreceptors for blue light, Examples of the plants that the present invention is applicable to include, but are not limited to, rice, wheat, barley, corn, bean, potato, adzuki bean, oats, millet, Arabidopsis thaliana, Chinese cabbage, radish, pepper, strawberry, tomato, water melon, cucumber, cabbage, oriental melon, bumpkin, onion, carrot, ginseng, tobacco, cotton, sesame, sugarcane, sugar beet, perilla, peanut, rape, apple, pear, jujube, peach, kiwi, grape, tangerine, persimmon, plum, apricot banana, rose, gladiolus, gerbera, carnation, chrysanthemum, lily, tulip, ryegrass, red clover, orchardgrass, alfalfa, tall rescue, and perennial ryegrass, with preference for Arabidopsis thaliana and plants belonging to brassicaceae. Among the plants belonging to brassicaceae are Chinese cabbage (Brassica campestris L. ssp. pekinensis or Brassica rapa L. ssp. pekinensis), rape, cabbage (Brassica oleracea L.), broccoli, cauliflower, kale, mustard, turnip or Brassica rapa, and radish.

As used herein, the term "plant" is understand to encompass plant cells, plant tissues, seeds, and those that can be developed adult plants, as well as adult plants.

### [Advantageous Effects]

According to the present invention, methods are provided for producing plants having enhanced or suppressed blue light perception ability.

### [Description of Drawings]

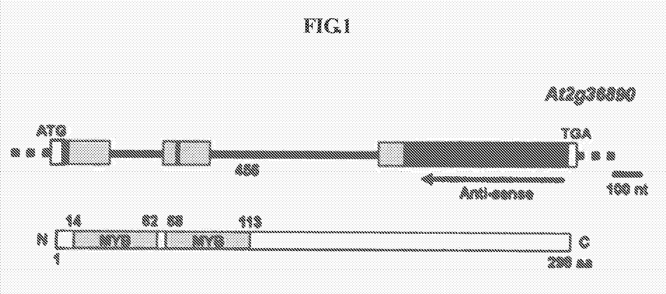

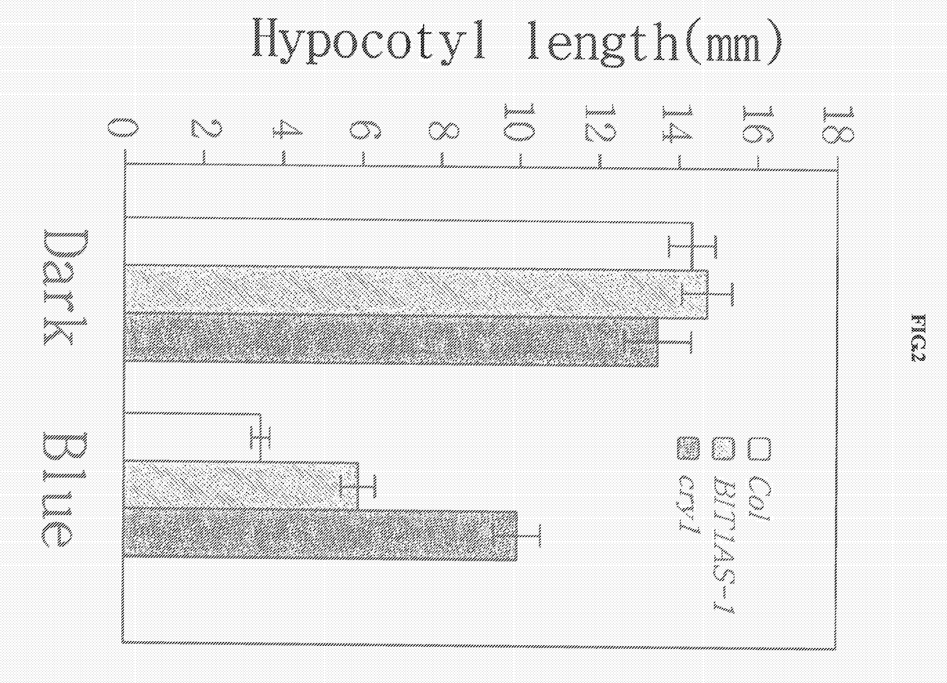

FIG. 1 is a schematic diagram showing a *BIT1* gene and its polypeptide.
   Gray Boxes: R3R4 TYPE MYB domain
   Thin line: Intron
   Arrow: used for preparing Antisense Line
FIG. 2 shows hypocotyl lengths of an Arabidopsis thaliana Columbia wild-type (Col), a blue light perception ability-suppressed Arabidopsis thaliana transfectant (BIT1 AS-1), and a crypthochrome variant (*cry1*) as a positive control after they were grown for 5 days in a dark room and under blue light at a fluence of 10 µmol m⁻² sec⁻¹.

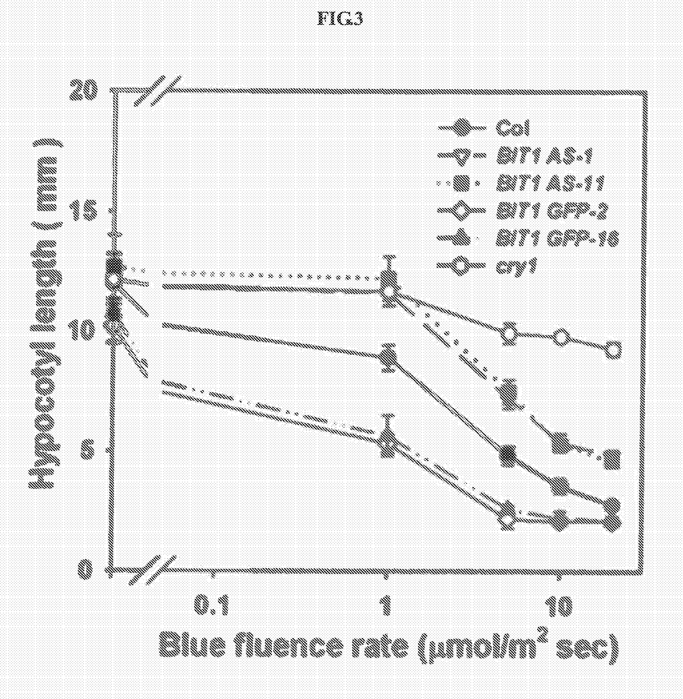

FIG. 3 shows hypocotyl lengths of an Arabidopsis thaliana Columbia wild-type, blue light signal transduction-modulated Arabidopsis thaliana transfectants (BIT1 AS-1, BIT1 AS-11, BIT1 GFP-2 and BIT1 GFP-16), and a crytochrome variant (*cry1*) as a positive control after they were grown under blue light at various intensities.

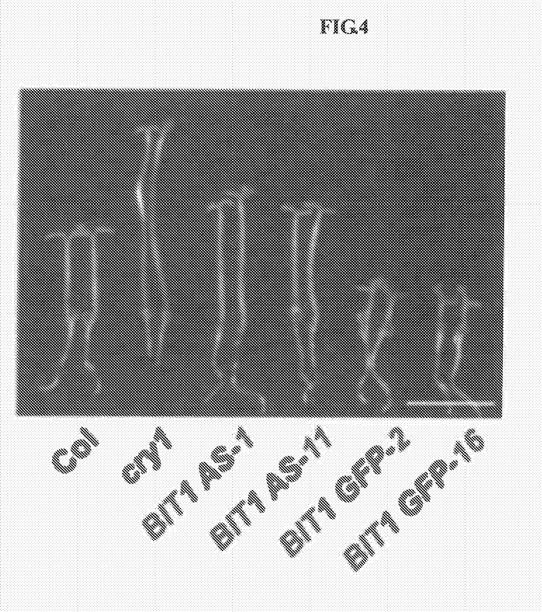

FIG. 4 is a photograph showing hypocotyl lengths of an Arabidopsis thaliana Columbia wild-type, blue light signal transduction-modulated Arabidopsis thaliana transfectants (*BIT1 AS-1, BIT1 AS-11, BIT1 GFP-2* and *BIT1 GFP-16*)*,* and a cryptochrome variant (*cry1*) as a positive control after they were incubated for 5 days under blue light at a fluence rate of 10 µmol m⁻² sec⁻¹.

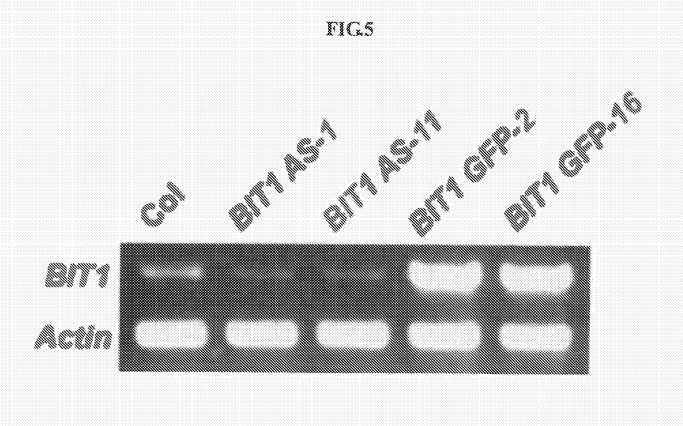

FIG. 5 shows the BIT1 gene expression levels in an Arabidopsis thaliana Columbia wild-type (Col) and blue light perception ability-enhanced or suppressed Arabidopsis thaliana transfectants (*BIT1 AS-1, BIT1 AS-11, BIT1 GFP-2* and *BIT1 GFP-16*) as measured by RT-PCR.

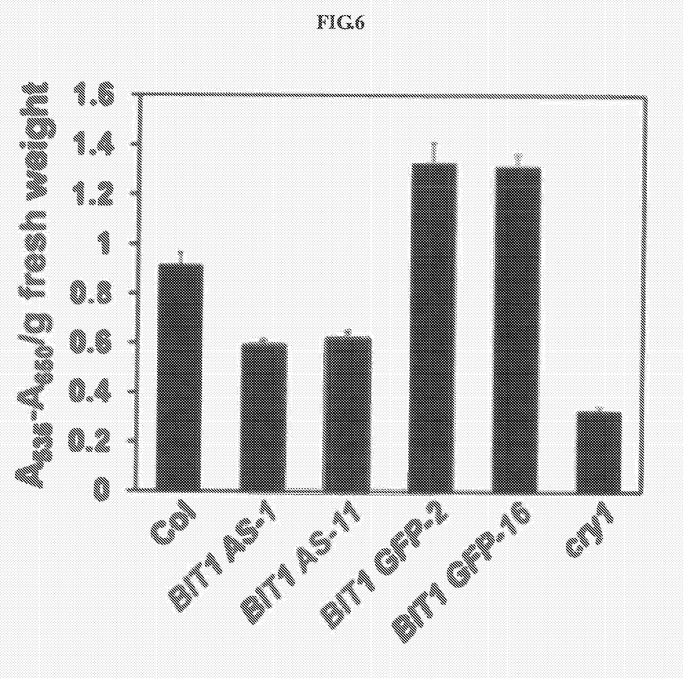

FIG. 6 shows the levels of anthocyanin synthesized in an Arabidopsis thaliana Columbia wild-type, blue light signal transduction-modulated Arabidopsis thaliana transfectants (BIT1 AS-1, BIT1 AS-11, BIT1 GFP-2 and BIT1 GFP-16), and a cryptochrome variant (*cry1*) as a positive control after they were grown under blue light.

### [Mode for Invention]

A better understanding of the present invention may be obtained through the following examples which are set forth to illustrate, but are not to be construed as limiting the present invention.

### <EXAMPLE 1> Preparation and Selection of Arabidopsis Thaliana Transformant Showing Suppressed Blue Light Signal Transduction

An Arabidopsis thaliana transformant showing suppressed blue light signal transduction was prepared and selected according to the method of Weigel et al. (Weigel et al., (2000) Plant Physiology, Vol 122 1003-1013)).

For this, a *BIT1* gene (having the nucleotide sequence of SEQ ID NO: 1 and the amino acid sequence of SEQ ID NO: 2, and the structures of its polynucleotide and polypeptide are depicted in FIG. 1) was employed. Its C-terminal region (546 base pairs from nt 349-894 in BIT1 cDNA) was closed in an antisense direction at a site between EcoRI and XhoI in a pNB96 vector (Jun et al., Planta (2002) 214: 668-674) to construct the recombinant expression vector pNB96::BIT1-C recombinant expression vector containing a BIT1 C-terminal region. Carrying a kanamycin-resistant gene, this recombinant expression vector pNB96::BIT1-C allowed its transformants to be readily selected, whether the transformants were derived from E. coli, Agrobacterium tumefaciens, or Arabidopsis thaliana. pNB96::BIT1-C was introduced into *Agrobacterium tumefaciens* strain (AGL1; Lazo et al., (1991) Biotechnology 9: 963-967) using electroporation, followed by the selection of a transformant resistant to kanamycin. Then, the transformant was transfected into Arabidopsis thaliana Columbia wild-type (Col-O(wt)) using a floral dipping method (Clough et al., Plant J (1998) 16: 735-743). An Arabidopsis thaliana transfectant was selected by culturing seeds from the transfected Arabidopsis thaliana in a medium containing 30 µg/ml. Thereafter, the selected Arabidopsis thaliana transfectant and the Arabidopsis thaliana Columbia wild-type (Col-O(wt)) were cultured at 22°C for 5 days under a source emitting blue light at a fluence rate of 10 µmol m⁻² sec⁻¹ with a light/dark cycle of 16/8 hrs in a growth chamber. The blue light perception ability of the plants was determined by comparing their hypocotyl lengths. As can be seen in FIG. 2, the Arabidopsis thaliana transfectant (*BIT1 AS*) showed a phenotype lower in blue light perception ability than did the Columbia wild-type (Col-O(wt)). A cryptochrome variant (*cry 1*) with blue light perception down-regulated was used as a positive control. There were no significant phenotype differences upon anthesis between the *BIT1* AS transfectant and the wild-type.

### <EXAMPLE 2> Production and Selection of Planet Transformant Showing Enhanced Blue Light Perception

To examine whether the phenotype of the Arabidopsis thaliana transfectant produced in Example 1 resulted from the suppression of *BIT1* gene expression, the phenotype of the Arabidopsis thaliana was observed when the BIT1 gene was overexpressed therein. First, a *BIT1* gene was closed at a site between EcoRI and XhoI in pCsVMV-GFP (containing a potential CsVMV (Cassava vein mosaic virus) promoter at position from -443 to +72 (Verdaguer et al., (1998) Plant Mol Biol. 37: 1055-67) to form a recombinant vector carrying the BIT1 gene, called pCsVMV-GFP::BIT1. The presence of a kanamycin-resistant gene in the recombinant vector pCsVMV-GFP::BIT1 allowed the selection of the E. coli or Agrobacterium tumefaciens transformed therewith. Also, the Arabidopsis thaliana transfectant harboring the vector can be readily selected because the vector carries a bygromycin-resistant gene.

The recombinant vector pCsVMV-GFP::BIT1 was introduced into Agrobacterium tumefaciens Agl1 (Lazo et al., (1991) Biotechnology 9: 963-967) by electroporation. Agrobacterium tumefaciens grown in the presence of kanamycin was transfected into Arabidopsis thaliana using a floral dipping method (Clough et al., (1998) Plant J. 16: 735-743). Arabidopsis thaliana transfectants were selected in a medium containing 15 µg/ml hygromycin. As can be seen in FIGS. 3 and 4, the Arabidopsis thaliana transfectants (*BIT1 GFP*) were observed to show enhanced blue light perception, unlike the control Arabidopsis thaliana. The *BIT1 GFP* transfectant was very small when it was a seedling, but grew bigger, and there were no significant phenotype differences at the time of anthesis between the transfectant and the wild-type.

### <EXAMPLE 3> RT-PCR Quantification of BIT1 Gene Expression in Plant Transfectants

To determine whether the Arabidopsis thaliana transfectant expressed the *BIT1* gene at a higher or lower level, RT-PCR was performed. First, total RNA was isolated from Arabidopsis thaliana Columbia wild-type (Col-O(wt)) and the Arabidopsis thaliana transfectants obtained in Examples 1 and 2 using an RNeasy plant mini kit (QIAGEN, USA) according to the manufacturer's instruction. cDNA was synthesized from 1 µg of each of the RNAs using ImProme-II reverse transcription system (Promega, USA) according to the manufacturer's instructions. PCR was performed under the following condition, with the cDNA serving as a template.

The total reaction volume was set to be 50 µL. In the PCR, the final concentrations of ingredients and the number and conditions of the reaction cycle are as follows.

### (Final Concentrations)

Template DNA: 1 µL
BIT1 forward primer (SEQ ID NO: 3): 0.2 µM
BIT1 reverse primer (SEQ ID NO: 4): 0.2 µM:
ACT forward primer (SEQ ID NO_{:} 5): 0.2 µM
ACT reverse primer (SEQ ID NO: 6) : 0.2 µM
dNTP mix: 0.8 mM
Tag buffer: 1x
Taq enzyme: 1 U

### (Number and Condition of reaction cycle)

30 cycles of 10 sec at 94°C, 30 sec at 56°C, 1 min at 72°C, followed by 10 min at 72°C.

As shown in FIG. 5, the gene was almost not expressed in the BIT1 antisense transfectant (*BIT1 AS*), compared to the wild-type (Col-0). On the other hand, a significant level of the gene was detected in the BIT1 overexpressed transfectant (*BIT1 GFP*).

### <EXAMPLE 4> Accumulation of Anthocyanin in plant Transfectant

To examine whether the Arabidopsis thaliana transfectant produced the blue light-induced anthocyanin in a larger or smaller amount than did the wild-type (Col-0), anthocyanin was quantified. First, seeds from Arabidopsis thaliana Columbia wild-type (Col-O(wt)) and the Arabidopsis thaliana transfectants obtained in Examples 1 and 2 were sterilized and subjected to cold treatment for 3 days before they were planted at a density of 50 seeds per 1/2 B5 medium. Exposure to white light for 12 hours induced germination, followed by culturing for 5 days under blue light. The plants grown under blue light were sampled, weighed, and immersed overnight at 4°C in 300 µl of 1% (v/v) hydrochloric acid in methanol according to the anthocyanin extraction method (Kim et al., (2003) Plant Cell, 15: 23992407). Relative anthocyanin concentrations were calculated by dividing absorbances at 530nm-657nm by each sample weight.

As can be seen in FIG. 6, the BIT1 antisense transfectant (*BIT1 AS*) accumulated anthocyanin at a lower level than did the wild-type (Col-0) whereas a significantly higher level of anthocyanin was accumulated in the BIT1-overexpressed transfectant (*BIT1 GFP*).

### [Sequence List Text]

Attached

<110>    POSTECH ACADEMY-INDUSTRY FOUNDATION

<120>    Method for Manufacturing a Plant Having Promoted or Supressed
         Capacity for Receiving Blue Light

<160>    6

<170>    KopatentIn 1.71

<210>    1
<211>    1188
<212>    DNA
<213>    Arabidopsis thaliana

<400>    1
atggttggtt ctaggaagcg ttcatcagcc aagaacagac ttgtttcttc ttcaactcgt
60

tcttctggga aggataaggt ttctgatgct cgtaaatatg gtgaagcctt ggtgggttca
120

aggatacgag tttggtggcc gatggacagc aaattctata agggtgtggt ggattcctat
180

gtctcttcca agaagaaaca tcgggttttc tatgaagatg gtgataaaga gaccttggat
240

ctgaagaagg agagatggga gttgattgaa gaagatgatg ctgaatctga atctgatgag
300

atctccctac aggaggaatc tgctggtgag agttctgaag gcactccaga accgcctaaa
360

gggaaaggga aggcatcttt aaaaggaagg acggatgaag ctatgcccaa aaagaaacag
420

aagatagatt cttcatcaaa gagcaaggcc aaagaggtgg agaagaaggc ctcaaagccc
480

gaaactgaga aaaatggcaa aatgggtgat attggtggga agattgtagc agcggcatca
540

cgcatgagtg aaaggtttcg gagcaagggg aatgtggatc aaaaagagac atcaaaggcc
600

tcgaaaaaac caaagatgtc ttcaaagttg accaagagga aacacactga tgaccaagat
660

gaagatgaag aagctggtga tgatattgat acttctagcg aagaggcgaa gcctaaggtg
720

ttaaaatcct gcaactccaa tgctgatgaa gttgctgaaa attcttctga tgaagatgag
780

cccaaggttc taaaaaccaa caactctaag gctgataaag atgaagatga agaggagaat
840

gaaacatctg atgatgaggc tgagcccaag gccctgaaat tgagcaactc caactctcat
900

aatggtgaaa acaattcatc tgatgatgag aaagagataa caatatcgaa gatcacctca
960

aaaaagatta agagtaatac cgctgatgaa gaaaatggtg acaatgaaga tggggaaaaa
1020

gctgttgatg aaatgtctga tggagagccg ttggtgagct tcttgaagaa gtcaggagag
1080

ggaattgatg cgaagaggaa aaaaatgaag gggaagaagg aggaagagga agaggaagga
1140

gaagaaaatg ctggaaaaga tacgaaagcg gaagaagcgt cgtcgtag
1188

<210>    2
<211>    395
<212>    PRT
<213>    Arabidopsis thaliana

<400>    2
Met Val Gly Ser Arg Lys Arg Ser Ser Ala Lys Asn Arg Leu Val Ser
1               5                   10                  15

Ser Ser Thr Arg Ser Ser Gly Lys Asp Lys Val Ser Asp Ala Arg Lys
            20                  25                  30

Thr Gly Glu Ala Leu Val Gly Ser Arg Ile Arg Val Trp Trp Pro Met
        35                  40                  45

Asp Ser Lys Phe Thr Lys Gly Val Val Asp Ser Tyr Val Ser Ser Lys
    50                  55                  60

Lys Lys His Arg Val Phe Tyr Glu Asp Gly Asp Lys Glu Thr Leu Asp
65                  70                  75                  80

Leu Lys Lys Glu Arg Trp Glu Leu Ile Glu Glu Asp Asp Ala Glu Ser
                85                  90                  95

Glu Ser Asp Glu Ile Ser Leu Gln Glu Glu Ser Ala Gly Glu Ser Ser
            100                 105                 110

Glu Gly Thr Pro Glu Pro Pro Lys Gly Lys Gly Lys Ala Ser Leu Lys
        115                 120                 125

Gly Arg Thr Asp Glu Ala Met Pro Lys Lys Lys Gln Lys Ile Asp Ser
    130                 135                 140

Ser Ser Lys Ser Lys Ala Lys Glu Val Glu Lys Lys Ala Ser Lys Pro
145                 150                 155                 160

Glu Thr Glu Lys Asn Gly Lys Met Gly Asp Ile Gly Gly Lys Ile Val
                165                 170                 175

Ala Ala Ala Ser Arg Met Ser Glu Arg Phe Arg Ser Lys Gly Asn Val
            180                 185                 190

Asp Gln Lys Glu Thr Ser Lys Ala Ser Lys Lys Pro Lys Met Ser Ser
        195                 200                 205

Lys Leu Thr Lys Arg Lys His Thr Asp Asp Gln Asp Glu Asp Glu Glu
    210                 215                 220

Ala Gly Asp Asp Ile Asp Thr Ser Ser Glu Glu Ala Lys Pro Lys Val
225                 230                 235                 240

Leu Lys Ser Cys Asn Ser Asn Ala Asp Glu Val Ala Glu Asn Ser Ser
                245                 250                 255

Asp Glu Asp Glu Pro Lys Val Leu Lys Thr Asn Asn Ser Lys Ala Asp
            260                 265                 270

Lys Asp Glu Asp Glu Glu Glu Asn Glu Thr Ser Asp Asp Glu Ala Glu
        275                 280                 285

Pro Lys Ala Leu Lys Leu Ser Asn Ser Asn Ser Asp Asn Gly Glu Asn
    290                 295                 300

Asn Ser Ser Asp Asp Glu Lys Glu Ile Thr Ile Ser Lys Ile Thr Ser
305                 310                 315                 320

Lys Lys Ile Lys Ser Asn Thr Ala Asp Glu Glu Asn Gly Asp Asn Glu
                325                 330                 335

Asp Gly Glu Lys Ala Val Asp Glu Met Ser Asp Gly Glu Pro Leu Val
            340                 345                 350

Ser Phe Leu Lys Lys Ser Pro Glu Gly Ile Asp Ala Lys Arg Lys Lys
        355                 360                 365

Met Lys Gly Lys Lys Glu Glu Glu Glu Glu Glu Gly Glu Glu Asn Ala
    370                 375                 380

Gly Lys Asp Thr Lys Ala Glu Glu Ala Ser Ser
385                 390                 395


<210>    3
<211>    29
<212>    DNA
<213>    Artificial Sequence

<220>
<223>    forward primer


<400>    3
gaattcatgg gtagggctcc atgttgtga
29


<210>    4
<211>    31
<212>    DNA
<213>    Artificial Sequence

<220>
<223>    reverse primer

<400>    4
ctcgaggtag tacaacatga acttatcctc c
31

<210>    5
<211>    24
<212>    DNA
<213>    Artificial Sequence

<220>
<223>    forward primer

<400>    5
ttccgctctt tctttccaag ctca
24

<210>    6
<211>    24
<212>    DNA
<213>    Artificial Sequence

<220>
<223>    reverse primer

<400>    6
aagaggcatc aattcgatca ctca
24

## Claims

1. A method for producing a plant having enhanced blue light perception ability, comprising:
(I) overexpressing a gene having a nucleotide sequence of SEQ ID NO: 1 or a nucleotide sequence similar to the nucleotide sequence of SEQ ID NO: 1; and
(II) selecting the plant which is enhanced in blue light perception ability as a result of the overexpression of the gene.

2. The method of claim 1, wherein the gene having a nucleotide sequence similar to the nucleotide sequence of SEQ ID NO: 1 encodes a polypeptide having an amino acid sequence of SEQ ID NO: 2.

3. The method of claim 1, wherein the overexpression step (I) comprises transforming a polynucleotide coding for a polypeptide having the amino acid sequence of SEQ ID NO: 2 into a plant.

4. The method of claim 1, wherein the overexpression step (I) comprises constructing a recombinant expression vector in which a polynucleotide coding for a polypeptide having the amino acid sequence of SEQ ID NO: 2 is operably linked to a regulatory nucleotide sequence, transforming the recombinant expression vector into Agrobacterium bacteria, and transfecting the transformed Agrobacterium bacteria into a plant.

5. A blue light perception ability-enhanced plant, produced using the method of any one of claims 1 to 4.

6. A method for producing a plant having suppressed blue light perception ability, comprising:
(I) down-regulating expression of a gene having a nucleotide sequence of SEQ ID NO: 1 or a nucleotide sequence similar to the nucleotide sequence of SEQ ID NO: 1; and
(II) selecting a plant which shows suppressed blue light perception ability as a result of the down-regulation.

7. The method of claim 6, wherein the gene having a nucleotide sequence similar to the nucleotide sequence of SEQ ID NO: 1 encodes a polypeptide having an amino acid sequence of SEQ ID NO: 2.

8. The method of claim 6, wherein the down-regulation step (I) comprises transforming the plant with an antisense nucleotide sequence complementary to one selected from the group consisting of the gene having the nucleotide sequence of SEQ ID NO: 1, mRNA of the gene having the nucleotide sequence of SEQ ID NO: 1, the gene having a nucleotide sequence similar to the nucleotide sequence of SEQ ID NO: 1, and mRNA of the gene having a nucleotide sequence similar to the nucleotide sequence of SEQ ID NO: 1.

9. The method of claim 8, wherein the transformation of the plant with the antisense nucleotide sequence comprises constructing an expression vector in which the antisense nucleotide sequence is operably linked to a regulatory nucleotide sequence, transforming the expression vector into Agrobacterium bacteria, and transfecting the transformed Agrobacterium bacteria info the plant.

10. The method of claim 6, wherein the down-regulation step (I) is carried out using one selected from the group consisting of gene deletion, gene insertion, T-DNA introduction, homologous recombination, transposon tagging, and siRNA (small interfering RNA).

11. A blue light perception ability-suppressed plant, produced using the method of any one of claims 6 to 10.

12. A method for producing an anthocyanin-hyperaccumulated plant, comprising:
(I) overexpressing a gene having a nucleotide sequence of SEQ ID NO: 1 or a nucleotide sequence similar to the nucleotide sequence of SEQ ID NO: 1; and
(II) selecting a planet in which anthocyanin is hyperaccumulated as a result of the overexpression of the gene.

13. The method of claim 12, wherein the gene having a nucleotide sequence similar to the nucleotide sequence of SEQ ID NO: 1 encodes a polypeptide having an amino acid sequence of SEQ ID NO: 2.

14. The method of claim 12, wherein the overexpression step (I), comprises transforming a polynucleotide coding for a polypeptide having the amino acid sequence of SEQ ID NO: 2 into a plant.

15. The method of claim 12, wherein the overexpression step (I) comprises constructing a recombinant expression vector in which a polynucleotide coding for a polypeptide having the amino acid sequence of SEQ ID NO: 2 is operable linked to a regulatory nucleotide sequence, transforming the recombinant expression vector into Agrobacterium bacteria, and transfecting the transformed Agrobacterium bacteria into a plant.

16. The anthocyanin-hyperaccumulated plant, produced using the method of any one of claims 12 to 15.

17. A method for selecting a transformed plant, comprising:
(I) transforming a plant with an expression vector containing a target gene, a polynucleotide having the nucleotide sequence of SEQ ID NO: 1 and a regulatory nucleotide sequence, said nucleotide sequence of SEQ ID NO: 1 encoding a protein causative of the hyperaccumulation of anthocyanin; and
(II) selecting a plant in which anthocyanin is hyperaccumulated.
